# EUROPEAN PATENT SPECIFICATION

(11) **EP 2 027 122 B1**
(45) Date of publication and mention of the grant of the patent: **10.03.2010**
(21) Application number: 07729315.7
(22) Date of filing: 21.05.2007
(51) Int. Cl.: C07D 471/08, A61K 31/551, A61P 25/00, A61P 25/28

(54) **NOVEL 1,4-DIAZA-BICYCLO[3.2.2]NONANE DERIVATIVES AND THEIR MEDICAL USE**
NEUARTIGE 1,4-DIAZABICYCLO[3.2.2]NONAN-DERIVATE UND DEREN MEDIZINISCHE VERWENDUNG
NOUVEAUX DÉRIVÉS DE 1,4-DIAZA-BICYCLO[3.2.2]NONANE ET LEUR UTILISATION MÉDICALE

(30) Priority: 23.05.2006 US 802529 P; 23.05.2006 DK 200600707
(43) Date of publication of application: 25.02.2009
(73) Proprietor: NeuroSearch A/S, 2750 Ballerup (DK)
(72) Inventor: PETERS, Dan, 2750 Ballerup (DK); OLSEN, Gunnar M., 2750 Ballerup (DK); NIELSEN, Elsebet Østergaard, 2750 Ballerup (DK); TIMMERMANN, Daniel, B., DK-2750 Ballerup (DK); LOECHEL, Steven, Charles, DK-2000 Frederiksberg (DK); CHRISTENSEN, Jeppe, Kejser, DK-2750 Ballerup (DK); DYHRING, Tino, DK-2750 Ballerup (DK)
(86) International application number: PCT/EP2007/054871
(87) International publication number: WO 2007/135122

(56) References cited:
- EP-A1- 1 231 212
- WO-A-2005/111038
- US-A1- 2003 130 305

## Description

### TECHNICAL FIELD

This invention relates to novel 1,4-diaza-bicyclo[3.2.2]nonane derivatives, which are found to be cholinergic ligands at the nicotinic acetylcholine receptors and modulators of the monoamine receptors and transporters. Due to their pharmacological profile the compounds of the invention may be useful for the treatment of diseases or disorders as diverse as those related to the cholinergic system of the central nervous system (CNS), the peripheral nervous system (PNS), diseases or disorders related to smooth muscle contraction, endocrine diseases or disorders, diseases or disorders related to neuro-degeneration, diseases or disorders related to inflammation, pain, and withdrawal symptoms caused by the termination of abuse of chemical substances.

### BACKGROUND ART

The endogenous cholinergic neurotransmitter, acetylcholine, exert its biological effect via two types of cholinergic receptors, the muscarinic Acetyl Choline Receptors (mAChR) and the nicotinic Acetyl Choline Receptors (nAChR).

As it is well established that muscarinic acetylcholine receptors dominate quantitatively over nicotinic acetylcholine receptors in the brain area important to memory and cognition, and much research aimed at the development of agents for the treatment of memory related disorders have focused on the synthesis of muscarinic acetylcholine receptor modulators.

Recently, however, an interest in the development of nAChR modulators has emerged. Several diseases are associated with degeneration of the cholinergic system i.e. senile dementia of the Alzheimer type, vascular dementia and cognitive impairment due to the organic brain damage disease related directly to alcoholism. Indeed several CNS disorders can be attributed to a cholinergic deficiency, a dopaminergic deficiency, an adrenergic deficiency or a serotonergic deficiency.

EP 1231212 describes pharmaceutical compositions comprising certain 1,4-diazabicyclo[3.2.2]nonane-carboxylic acid derivatives useful as nicotinic α7 receptor agonists.

### SUMMARY OF THE INVENTION

The present invention is devoted to the provision novel modulators of the nicotinic and/or of the monoamine receptors, which modulators are useful for the treatment of diseases or disorders related to the cholinergic receptors, and in particular the nicotinic acetylcholine receptor (nAChR), the serotonin receptor (5-HTR), the dopamine receptor (DAR) and the norepinephrine receptor (NER), and of the biogenic amine transporters for serotonin (5-HT), dopamine (DA) and norepinephrine (NE).

Due to their pharmacological profile the compounds of the invention may be useful for the treatment of diseases or disorders as diverse as those related to the cholinergic system of the central nervous system (CNS), the peripheral nervous system (PNS), diseases or disorders related to smooth muscle contraction, endocrine diseases or disorders, diseases or disorders related to neuro-degeneration, diseases or disorders related to inflammation, pain, and withdrawal symptoms caused by the termination of abuse of chemical substances.

The compounds of the invention may also be useful as diagnostic tools or monitoring agents in various diagnostic methods, and in particular for *in vivo* receptor imaging (neuroimaging), and they may be used in labelled or unlabelled form.

In its first aspect the invention provides novel 1,4-diaza-bicyclo[3.2.2]nonane derivatives of Formula I 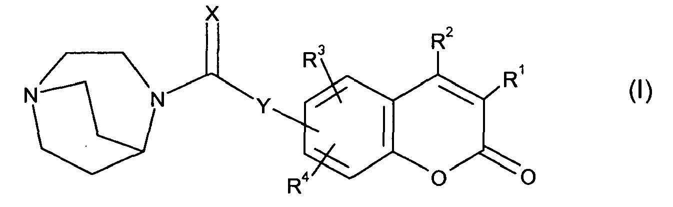 or a pharmaceutically acceptable salt thereof, wherein
X represents O;
Y represents O;
R¹ and R², independently of each other, represent hydrogen, halo or trifluoromethyl; and

R³ and R⁴ both represent hydrogen.

In its second aspect the invention provides pharmaceutical compositions comprising a therapeutically effective amount of the 1,4-diaza-bicyclo[3.2.2]nonane derivatives of the invention, or a pharmaceutically-acceptable addition salt thereof together with at least one pharmaceutically-acceptable carrier or diluent.

In a further aspect the invention relates to the use of the 1,4-diaza-bicyclo[3.2.2]nonane derivatives of the invention, or a pharmaceutically-acceptable addition salt thereof, for the manufacture of a pharmaceutical composition/medicament for the treatment, prevention or alleviation of a disease or a disorder or a condition of a mammal, including a human, which disease, disorder or condition is responsive to modulation of cholinergic receptors and/or monoamine receptors.

Other objects of the invention will be apparent to the person skilled in the art from the following detailed description and examples.

### DETAILED DISCLOSURE OF THE INVENTION

### 1,4-Diaza-bicyclo[3.2.2]nonane Derivatives

In a first aspect novel 1,4-diaza-bicyclo[3.2.2]nonane derivatives are provided. The 1,4-diaza-bicyclo[3.2.2]nonane derivatives of the invention may be represented by the general Formula I 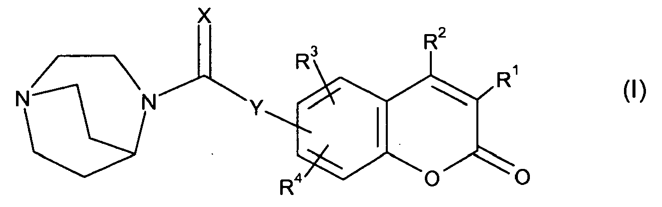 or a pharmaceutically acceptable salt thereof, wherein
X represents O;
Y represents O;
R¹ and R², independently of each other, represent hydrogen, halo or trifluoromethyl; and
R³ and R⁴ both represent hydrogen.

In a more preferred embodiment one of R¹ and R² represents hydrogen, halo or trifluoromethyl; and the other of R¹ and R², together with R³ and R⁴ all represent hydrogen.

In another more preferred embodiment R¹, R², R³ and R⁴ all represent hydrogen.

In a third more preferred embodiment R¹ represents halo; and R², R³ and R⁴ all represent hydrogen.

In a most preferred embodiment the 1,4-diaza-bicyclo[3.2.2]nonane derivative of the invention is
1,4-Diaza-bicyclo[3.2.2]nonane-4-carboxylic acid 2-oxo-2*H*-chromen-7-yl ester;
1,4-Diaza-bicyclo[3.2.2]nonane-4-carboxylic acid 2-oxo-2*H*-chromen-6-yl ester; or
1,4-Diaza-bicyclo[3.2.2]nonane-4-carboxylic acid 3-bromo-2-oxo-2*H-*chromen-7-yl ester;
or a pharmaceutically acceptable salt thereof.

Any combination of two or more of the embodiments described herein is considered within the scope of the present invention.

### Definition of Substituents

In the context of this invention halo represents fluoro, chloro, bromo or iodo. Thus a trihalomethyl group represents e.g. a trifluoromethyl group, a trichloromethyl group, and similar trihalo-substituted methyl groups.

### Pharmaceutically Acceptable Salts

The 1,4-diaza-bicyclo[3.2.2]nonane derivative of the invention may be provided in any form suitable for the intended administration. Suitable forms include pharmaceutically (i.e. physiologically) acceptable salts of the chemical compound of the invention.

Examples of pharmaceutically acceptable addition salts include, without limitation, the non-toxic inorganic and organic acid addition salts such as the hydrochloride, the hydrobromide, the nitrate, the perchlorate, the phosphate, the sulphate, the formate, the acetate, the aconate, the ascorbate, the benzenesulphonate, the benzoate, the cinnamate, the citrate, the embonate, the enantate, the fumarate, the glutamate, the glycolate, the lactate, the maleate, the malonate, the mandelate, the methanesulphonate, the naphthalene-2-sulphonate derived, the phthalate, the salicylate, the sorbate, the stearate, the succinate, the tartrate, the toluene-p-sulphonate, and the like. Such salts may be formed by procedures well known and described in the art.

Metal salts of a chemical compound of the invention include alkali metal salts, such as the sodium salt of a chemical compound of the invention containing a carboxy group.

In the context of this invention the "onium salts" of N-containing compounds are also contemplated as pharmaceutically acceptable salts. Preferred "onium salts" include the alkyl-onium salts, the cycloalkyl-onium salts, and the cycloalkylalkyl-onium salts.

### Labelled Compounds

The compounds of the invention may be used in their labelled or unlabelled form. In the context of this invention the labelled compound has one or more atoms replaced by an atom having an atomic mass or mass number different from the atomic mass or mass number usually found in nature. The labelling will allow easy quantitative detection of said compound.

The labelled compounds of the invention may be useful as diagnostic tools, radio tracers, or monitoring agents in various diagnostic methods, and for *in vivo* receptor imaging.

The labelled isomer of the invention preferably contains at least one radionuclide as a label. Positron emitting radionuclides are all candidates for usage. In the context of this invention the radionuclide is preferably selected from ²H (deuterium), ³H (tritium), ¹³C, ¹⁴C, ¹³¹I, ¹²⁵I, ¹²³I, and ¹⁸F.

The physical method for detecting the labelled isomer of the present invention may be selected from Position Emission Tomography (PET), Single Photon Imaging Computed Tomography (SPECT), Magnetic Resonance Spectroscopy (MRS), Magnetic Resonance Imaging (MRI), and Computed Axial X-ray Tomography (CAT), and combinations thereof.

### Methods of Producing 1,4-Diaza-bicyclo[3.2.2]nonane Derivatives

The 1,4-diaza-bicyclo[3.2.2]nonane derivative of the invention may be prepared by conventional methods for chemical synthesis, e.g. those described in the working examples. The starting materials for the processes described in the present application are known or may readily be prepared by conventional methods from commercially available chemicals.

Also one compound of the invention can be converted to another compound of the invention using conventional methods.

The end products of the reactions described herein may be isolated by conventional techniques, e.g. by extraction, crystallisation, distillation, chromatography, etc.

### Biological Activity

The present invention is devoted to the provision novel ligands and modulators of the nicotinic receptors, which ligands and modulators are useful for the treatment of diseases or disorders related to the cholinergic receptors, and in particular the nicotinic acetylcholine receptor (nAChR). Preferred compounds of the invention show a pronounced nicotinic acetylcholine α7 receptor subtype selectivity.

Due to their pharmacological profile the compounds of the invention may be useful for the treatment of diseases or conditions as diverse as CNS related diseases, PNS related diseases, diseases related to smooth muscle contraction, endocrine disorders, diseases related to neuro-degeneration, diseases related to inflammation, pain, and withdrawal symptoms caused by the termination of abuse of chemical substances.

In a preferred embodiment the compounds of the present invention may be useful for the treatment, prevention or alleviation of a cognitive disorder, learning deficit, memory deficits and dysfunction, Down's syndrome, Alzheimer's disease, attention deficit, attention deficit hyperactivity disorder (ADHD), Tourette's syndrome, psychosis, depression, Bipolar Disorder, mania, manic depression, schizophrenia, cognitive or attention deficits related to schizophrenia, obsessive compulsive disorders (OCD), panic disorders, eating disorders such as anorexia nervosa, bulimia and obesity, narcolepsy, nociception, AIDS-dementia, senile dementia, autism, Parkinson's disease, Huntington's disease, Amyotrophic Lateral Sclerosis, anxiety, non-OCD anxiety disorders, convulsive disorders, epilepsy, neurodegenerative disorders, transient anoxia, induced neuro-degeneration, neuropathy, diabetic neuropathy, periferic dyslexia, tardive dyskinesia, hyperkinesia, mild pain, moderate or severe pain, pain of acute, chronic or recurrent character, pain caused by migraine, postoperative pain, phantom limb pain, inflammatory pain, neuropathic pain, chronic headache, central pain, pain related to diabetic neuropathy, to post therapeutic neuralgia, or to peripheral nerve injury, bulimia, post-traumatic syndrome, social phobia, sleeping disorders, pseudodementia, Ganser's syndrome, pre-menstrual syndrome, late luteal phase syndrome, fibromyalgia, chronic fatigue syndrome, mutism, trichotillomania, jet-lag, arrhythmias, smooth muscle contractions, angina pectoris, premature labour, diarrhoea, asthma, tardive dyskinesia, hyperkinesia, premature ejaculation, erectile difficulty, hypertension, inflammatory disorders, inflammatory skin disorders, acne, rosacea, Chron's disease, inflammatory bowel disease, ulcerative colitis, diarrhoea, or withdrawal symptoms caused by termination of use of addictive substances, including nicotine containing products such as tobacco, opioids such as heroin, cocaine and morphine, benzodiazepines and benzodiazepine-like drugs, and alcohol.

In a more preferred embodiment the compounds of the invention may be useful for the treatment, prevention or alleviation of pain, mild or moderate or severe pain, pain of acute, chronic or recurrent character, pain caused by migraine, postoperative pain, phantom limb pain, inflammatory pain, neuropathic pain, chronic headache, central pain, pain related to diabetic neuropathy, to post therapeutic neuralgia, or to peripheral nerve injury.

In an even more preferred embodiment the compounds of the invention may be useful for the treatment, prevention or alleviation of diseases, disorders or conditions associated with smooth muscle contractions, convulsive disorders, angina pectoris, premature labour, convulsions, diarrhoea, asthma, epilepsy, tardive dyskinesia, hyperkinesia, premature ejaculation, or erectile difficulty.

In a still more preferred embodiment the compounds of the invention may be useful for the treatment, prevention or alleviation of a neurodegenerative disorder, transient anoxia, or induced neuro-degeneration.

In a yet more preferred embodiment the compounds of the invention may be useful for the treatment, prevention or alleviation of an inflammatory disorder, inflammatory skin disorder, acne, rosacea, Chron's disease, inflammatory bowel disease, ulcerative colitis, or diarrhoea.

In a further preferred embodiment the compounds of the invention may be useful for the treatment, prevention or alleviation of diabetic neuropathy, schizophrenia, cognitive or attentional deficits related to schizophrenia, or depression.

Finally the compounds of the invention may be useful for the treatment of withdrawal symptoms caused by termination of use of addictive substances. Such addictive substances include nicotine containing products such as tobacco, opioids such as heroin, cocaine and morphine, benzodiazepines, benzodiazepine-like drugs, and alcohol. Withdrawal from addictive substances is in general a traumatic experience characterised by anxiety and frustration, anger, anxiety, difficulties in concentrating, restlessness, decreased heart rate and increased appetite and weight gain.

In this context "treatment" covers treatment, prevention, prophylactics and alleviation of withdrawal symptoms and abstinence as well as treatment resulting in a voluntary diminished intake of the addictive substance.

In another aspect, the compounds of the invention are used as diagnostic agents, e.g. for the identification and localisation of nicotinic receptors in various tissues.

It is at present contemplated that a suitable dosage of the active pharmaceutical ingredient (API) is within the range of from about 0.1 to about 1000 mg API per day, more preferred of from about 10 to about 500 mg API per day, most preferred of from about 30 to about 100 mg API per day, dependent, however, upon the exact mode of administration, the form in which it is administered, the indication considered, the subject and in particular the body weight of the subject involved, and further the preference and experience of the physician or veterinarian in charge.

Preferred compounds of the invention show a biological activity in the sub-micromolar and micromolar range, i.e. of from below 1 to about 100 µM.

### Pharmaceutical Compositions

In another aspect the invention provides novel pharmaceutical compositions comprising a therapeutically effective amount of the 1,4-diaza-bicyclo[3.2.2]nonane derivatives, derivative of the invention.

While a chemical compound of the invention for use in therapy may be administered in the form of the raw chemical compound, it is preferred to introduce the active ingredient, optionally in the form of a physiologically acceptable salt, in a pharmaceutical composition together with one or more adjuvants, excipients, carriers, buffers, diluents, and/or other customary pharmaceutical auxiliaries.

In a preferred embodiment, the invention provides pharmaceutical compositions comprising the 1,4-diaza-bicyclo[3.2.2]nonane derivative of the invention, or a pharmaceutically acceptable salt or derivative thereof, together with one or more pharmaceutically acceptable carriers therefore, and, optionally, other therapeutic and/or prophylactic ingredients, know and used in the art. The carrier(s) must be "acceptable" in the sense of being compatible with the other ingredients of the formulation and not harmful to the recipient thereof.

The pharmaceutical composition of the invention may be administered by any convenient route, which suits the desired therapy. Preferred routes of administration include oral administration, in particular in tablet, in capsule, in dragé, in powder, or in liquid form, and parenteral administration, in particular cutaneous, subcutaneous, intramuscular, or intravenous injection. The pharmaceutical composition of the invention can be manufactured by any skilled person by use of standard methods and conventional techniques appropriate to the desired formulation. When desired, compositions adapted to give sustained release of the active ingredient may be employed.

Further details on techniques for formulation and administration may be found in the latest edition of Remington's Pharmaceutical Sciences (Maack Publishing Co., Easton, PA).

The actual dosage depends on the nature and severity of the disease being treated, and is within the discretion of the physician, and may be varied by titration of the dosage to the particular circumstances of this invention to produce the desired therapeutic effect. However, it is presently contemplated that pharmaceutical compositions containing of from about 0.1 to about 500 mg of active ingredient per individual dose, preferably of from about 1 to about 100 mg, most preferred of from about 1 to about 10 mg, are suitable for therapeutic treatments.

The active ingredient may be administered in one or several doses per day. A satisfactory result can, in certain instances, be obtained at a dosage as low as 0.1 µg/kg i.v. and 1 µg/kg p.o. The upper limit of the dosage range is presently considered to be about 10 mg/kg i.v. and 100 mg/kg p.o. Preferred ranges are from about 0.1 µg/kg to about 10 mg/kg/day i.v., and from about 1 µg/kg to about 100 mg/kg/day p.o.

### EXAMPLES

The invention is further illustrated with reference to the following examples, which are not intended to be in any way limiting to the scope of the invention as claimed.

### Example 1

### Preparatory Example

All reactions involving air sensitive reagents or intermediates were performed under nitrogen and in anhydrous solvents. Magnesium sulfate was used as drying agent in the workup-procedures and solvents were evaporated under reduced pressure.

### 1,4-Diazabicyclo[3.2.2]nonane (Intermediate compound)

The title compound was prepared according to J. Med. Chem. 1993 36 2311-2320 (and according to a slightly modified method below).

### 1,4-Diazabicyclo[3.2.2]nonane (Intermediate compound)

To the solution of 1,4-diazabicyclo[3.2.2]nonan-3-one (15.8 g; 113 mmol) in absolute dioxane (130 ml) LiAlH₄ (4.9 g; 130 mmol) was added under argon. The mixture was refluxed for 6 h and then allowed to reach room temperature. To the reaction mixture water (5 ml in 10 ml of dioxane) was added by drops, the mixture was stirred for 0.5 hour and then filtered off via glass filter. The solvent was evaporated and the residue was distilled using Kugelrohr apparatus at 90°C (0.1 mbar) to yield 1,4-diazabicyclo[3.2.2]nonane (11.1 g; 78%) as colourless hygroscopic material.

### 1,4-Diazabicyclo[3.2.2]nonan-3-one (intermediate compound)

To the solution of 3-quinuclidinone hydrochloride (45 g; 278 mmol) in 90 ml of water hydroxylamine hydrochloride (21 g; 302 mmol) and sodium acetate (CH₃COOHx3H₂O; 83 g; 610 mmol) were added, the mixture was stirred at 70°C for 1 hour and then cooled to 0°C. The separated crystalline material was filtered off (without washing) and dried *in vacuo* to yield 40.0 g of oxime.

The 3-quinuclidinone oxime (40.0 g) was added during 2 hours by small portions to preheated to 120°C polyphosphoric acid (190 g). The temperature of the solution during the reaction was kept at 130°C. After addition of all oxime the solution was stirred for 20 minutes at the same temperature, then transferred to an enamelled vessel and allowed to reach room temperature. The acidic mixture was neutralized by a solution of potassium carbonate (500 g in 300 ml of water), transferred into 2000 ml flask, diluted with 300 ml of water and extracted with chloroform (3 x 600 ml). The combined organic extracts were dried with sodium sulphate, the solvent evaporated and the solid residue dried up *in vacuo* to yield 30.0 g (77%) of the mixture of lactams.

Crystallization of the obtained mixture from 1,4-dioxane (220 ml) gave 15.8 g (40.5%) of 1,4-diazabicyclo[3.2.2]nonan-3-one as colourless large crystals with mp. 211-212°C.

### Method A

### 1,4-Diaza-bicyclo[3.2.2]nonane-4-carboxylic acid 2-oxo-2H-chromen-7-yl ester free base (Compound A1)

Phosgen, 20% in toluene (3.05 g, 30.8 mmol) was solved in anhydrous dichloromethane (15 ml) at 0°C. A mixture of 7-hydroxy-chromen-2-one (1.00 g, 6.17 mmol), pyridine (0.63 g, 8.01 mmol), and dichloromethane (40 ml) was added to the mixture at 0°C. The mixture was stirred for 30 minutes at 0°C. The mixture was allowed reach room-temperature and was stirred over-night. The reaction-mixture was evaporated and was then co-evaporated with toluene (25 ml). The crude chlorocarbonylated intermediate, 1,4-diazabicyclo[3.2.2]nonane (0.78 g 6.17 mmol) and 1,2-dimethoxyethane (20 ml) was stirred at room-temperature for 20 hours. The mixture was filtered. The free base was obtained by adding a mixture of sodium methoxide (2 eq.), methanol (20 ml) and silica gel (5 g), followed by evaporation. The crude mixture was separated by column chromatography on silica gel with dichloromethane, 10% methanol and 1% aqueous ammonia as solvent gave the title compound as a solid. Yield 140 mg, 7%. LC-ESI-HRMS of [M+H]+ shows 315.1332 Da. Calc. 315.134483 Da, dev. -4.1 ppm.

### 1,4-Diaza-bicyclo[3.2.2]nonane-4-carboxylic acid 2-oxo-2H-chromen-6-yl ester free base (Compound A2)

Was prepared according to Method A from 6-hydroxy-chromen-2-one. LC-ESI-HRMS of [M+H]+ shows 315.1336 Da. Calc. 315.134483 Da, dev. -2.8 ppm.

### Method B

### 1,4-Diaza-bicyclo[3.2.2]nonane-4-carboxylic acid 3-bromo-2-oxo-2H-chromen-7-yl ester hydrobromide salt (Compound B1)

1,4-Diaza-bicyclo[3.2.2]nonane-4-carboxylic acid 2-oxo-2*H*-chromen-7-yl ester (0.75 g, 2.39 mmol), sodium acetate (1.17 g, 14.3 mmol), acetic acid (25 ml) and bromine (0.84 g, 5.23 mmol) was stirred at room temperature for 15 h. Water (25 ml ) was added, the mixture was stirred and filtered. The crystalline product was washed with water, methanol and diethylether. Yield 550 mg (42%). LC-ESI-HRMS of [M+H]+ shows 393.0464 Da. Calc. 393.044996 Da, dev. 3.6 ppm.

### 1,4-Diaza-bicyclo[3.2.2]nonane-4-carboxylic acid 3-bromo-2-oxo-2H-chromen-6-yl ester (Compound B2)

Is prepared according to Method B.

### 1,4-Diaza-bicyclo[3.2.2]nonane-4-carboxylic acid 3-chloro-2-oxo-2H-chromen-7-yl ester (Compound B3)

Is prepared according to Method B using chlorine, solved in acetic acid.

### 1,4-Diaza-bicyclo[3.2.2]nonane-4-carboxylic acid 3-chloro-2-oxo-2H-chromen-6-yl ester (Compound B4)

Is prepared according to Method B using chlorine, solved in acetic acid.

## Claims

1. A 1,4-diaza-bicycto[3.2.2]nonane derivative represented by Formula I 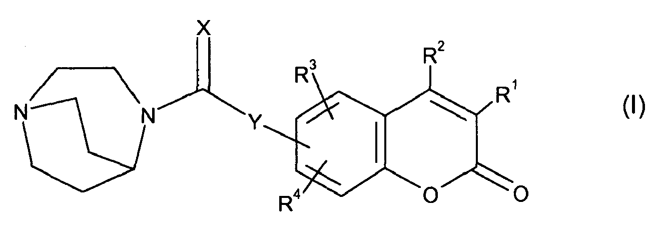 or a pharmaceutically acceptable salt thereof, wherein
X represents O;
Y represents O;
R¹ and R², independently of each other, represent hydrogen, halo or trifluoromethyl; and
R³ and R⁴ both represent hydrogen.

2. The 1,4-diaza-bicyclo[3.2.2]nonane derivative of claim 1, wherein
one of R¹ and R² represents hydrogen, halo or trifluoromethyl; and
the other of R¹ and R², together with R³ and R⁴ all represent hydrogen.

3. The 1,4-diaza-bicyclo[3.2.2]nonane derivative of claim 1, wherein
R¹ represents halo; and
R², R³ and R⁴ all represent hydrogen.

4. The 1,4-diaza-bicyclo[3.2.2]nonane derivative of claim 1, wherein R¹, R², R³ and R⁴ all represent hydrogen.

5. The 1,4-diaza-bicyclo[3.2.2]nonane derivative of claim 1, which is
1,4-Diaza-bicyclo[3.2.2]nonane-4-carboxylic acid 2-oxo-2*H*-chromen-7-yl ester;
1,4-Diaza-bicyclo[3.2.2]nonane-4-carboxylic acid 2-oxo-2*H*-chromen-6-yl ester; or
1,4-Diaza-bicyclo[3.2.2]nonane-4-carboxylic acid 3-bromo-2-oxo-2*H*-chromen-7-yl ester;
or a pharmaceutically acceptable salt thereof.

6. A pharmaceutical composition comprising a therapeutically effective amount of the 1,4-diaza-bicyclo[3.2.2]nonane derivative of any one of claims 1-5, or a pharmaceutically-acceptable addition salt thereof, together with at least one pharmaceutically-acceptable carrier or diluent.

7. Use of the 1,4-diaza-bicyclo[3.2.2]nonane derivative of any one of claims 1-5, or a pharmaceutically-acceptable addition salt thereof, for the manufacture of a pharmaceutical composition/medicament for the treatment, prevention or alleviation of a disease or a disorder or a condition of a mammal, including a human, which disease, disorder or condition is a cognitive disorder, teaming deficit, memory deficit or dysfunction, Down's syndrome, Alzheimer's disease, attention deficit, attention deficit hyperactivity disorder (ADHD), Tourette's syndrome, psychosis, depression, Bipolar Disorder, mania, manic depression, schizophrenia, cognitive or attention deficits related to schizophrenia, obsessive compulsive disorder (OCD), panic disorder, an eating disorder, anorexia nervosa, bulimia, obesity, narcolepsy, nociception, AIDS-dementia, senile dementia, autism, Parkinson's disease, Huntington's disease, Amyotrophic Lateral Sclerosis, anxiety, non-OCD anxiety disorder, convulsive disorder, epilepsy, neurodegenerative disorder, transient anoxia, induced neuro-degeneration, neuropathy, diabetic neuropathy, periferal dyslexia, tardive dyskinesia, hyperkinesia, mild pain, moderate or severe pain, pain of acute, chronic or recurrent character, pain caused by migraine, postoperative pain, phantom limb pain, inflammatory pain, neuropathic pain, chronic headache, central pain, pain related to diabetic neuropathy, to postherpetic neuralgia, or to peripheral nerve injury, post-traumatic syndrome, social phobia, sleeping disorder, pseudodementia, Ganser's syndrome, pre-menstrual syndrome, late luteal phase syndrome, fibromyalgia, chronic fatigue syndrome, mutism, trichotillomania, jet-lag, arrhythmias, smooth muscle contractions, angina pectoris, premature labour, diarrhoea, asthma, tardive dyskinesia, hyperkinesia, premature ejaculation, erectile difficulty, hypertension, inflammatory disorder, inflammatory skin disorder, acne, rosacea, Crohn's disease, inflammatory bowel disease, ulcerative colitis, diarrhoea, or withdrawal symptoms caused by termination of use of tobacco, opioids, heroin, cocaine or morphine, benzodiazepines and benzodiazepine-like drugs, and alcohol.

8. The 1,4-diaza-bicyclo[3.2.2]nonane derivative of any one of claims 1-5, or a pharmaceutically-acceptable addition salt thereof, for use as a pharmaceutical composition/medicament.

## Patentansprüche

1. 1,4-Diaza-bicyclo[3.2.2]nonan-Derivat, das durch Formel I wiedergegeben ist 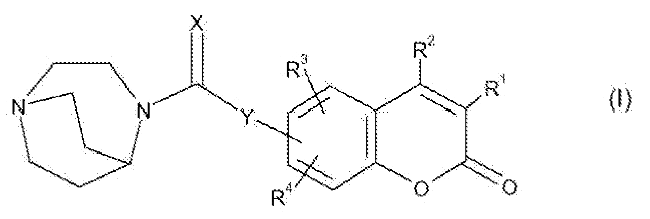 oder ein pharmazeutisch verträgliches Salz davon, wobei
X O bedeutet;
Y O bedeutet;
R¹ und R² unabhängig voneinander Wasserstoff, Halogen oder Trifluormethyl bedeuten; und
R³ und R⁴ beide Wasserstoff bedeuten.

2. 1,4-Diaza-bicyclo[3.2.2]nonan-Derivat nach Anspruch 1, wobei eines von R¹ und R² Wasserstoff, Halogen oder Trifluormethyl bedeutet; und das andere von R¹ und R², zusammen mit R³ und R⁴ alle Wasserstoff bedeuten.

3. 1,4-Diaza-bicyclo[3.2.2]nonan-Derivat nach Anspruch 1, wobei
R¹ Halogen bedeutet; und
R², R³ und R⁴ alle Wasserstoff bedeuten.

4. 1,4-Diaza-bicyclo[3.2.2]nonan-Derivat nach Anspruch 1, wobei R¹, R², R³ und R⁴ alle Wasserstoff bedeuten.

5. 1,4-Diaza-bicyclo[3.2.2]nonan-Derivat nach Anspruch 1, welches
1,4-Diaza-bicyclo[3.2.2]nonan-4-carbonsäure-2-oxo-2*H*-chromen-7-yl-ester;
1,4-Diaza-bicyclo[3.2.2]nonan-4-carbonsäure-2-oxo-2*H*-chromen-6-yl-ester; oder
1,4-Diaza-bicyclo[3.2.2]nonan-4-carbonsäure-3-brom-2-oxo-2*H*-chromen-7-yl-ester ist;
oder ein pharmazeutisch verträgliches Salz davon.

6. Pharmazeutische Zusammensetzung, umfassend eine therapeutisch wirksame Menge des 1,4-Diaza-bicyclo[3.2.2]nonan-Derivats nach einem der Ansprüche 1-5, oder eines pharmazeutisch verträglichen Additionssalzes davon, zusammen mit wenigstens einem pharmazeutisch verträglichen Träger oder Verdünnungsmittel.

7. Verwendung des 1,4-Diaza-bicyclo[3.2.2]nonan-Derivats nach einem der Ansprüche 1-5, oder eines pharmazeutisch verträglichen Additionssalzes davon, für die Herstellung einer pharmazeutischen Zusammensetzung/eines Medikaments für die Behandlung, Verhütung oder Linderung einer Krankheit oder einer Störung oder eines Zustands eines Säugers, einschließlich eines Menschen, wobei es sich bei dieser Krankheit, dieser Störung oder diesem Zustand um eine kognitive Störung, ein Lerndefizit, ein Gedächtnisdefizit oder eine Gedächtnisdysfunktion, das Down-Syndrom, die Alzheimer-Krankheit, ein Aufmerksamkeitsdefizit, eine Aufmerksamkeitsdefizit-Hyperaktivitätsstörung (ADHD), das Tourette-Syndrom, eine Psychose, Depression, bipolare Störung, Manie, manische Depression, Schizophrenie, kognitive oder Aufmerksamkeitsdefizite in Verbindung mit Schizophrenie, eine Zwangsstörung (OCD), eine Panikstörung, eine Essstörung, Anorexia nervosa, Bulimie, Fettleibigkeit, Narkolepsie, Nozizeption, AIDS-Demenz, senile Demenz, Autismus, die Parkinson-Krankheit, Chorea Huntington, amyotrophe Lateralsklerose, Angst, eine Nicht-OCD-Angststörung, eine konvulsive Störung, Epilepsie, eine neurodegenerative Störung, vorübergehende Anoxie, induzierte Neurodegeneration, Neuropathie, diabetische Neuropathie, periphere Dyslexie, tardive Dyskinesie, Hyperkinesie, milde Schmerzen, mäßige oder starke Schmerzen, Schmerzen von akutem, chronischem oder wiederkehrendem Charakter, durch Migräne verursachte Schmerzen, postoperative Schmerzen, Phantomschmerzen, entzündliche Schmerzen, neuropathische Schmerzen, chronische Kopfschmerzen, zentrale Schmerzen, Schmerzen in Verbindung mit diabetischer Neuropathie, mit postherpetischer Neuralgie oder mit peripherer Nervenverletzung, das posttraumatische Syndrom, eine Sozialphobie, eine Schlafstörung, Pseudodemenz, das Ganser-Syndrom, das prämenstruelle Syndrom, das Syndrom der späten Lutealphase, Fibromyalgie, das chronische Ermüdungssyndrom, Mutismus, Trichotillomanie, Jetlag, Arrhythmien, Kontraktionen eines glatten Muskels, Angina pectoris, eine Frühgeburt, Diarrhö, Asthma, tardive Dyskinesie, Hyperkinesie, vorzeitige Ejakulation, Erektionsschwierigkeiten, Hochdruck, eine entzündliche Störung, eine entzündliche Hautstörung, Akne, Rosacea, Morbus Crohn, eine entzündliche Darmerkrankung, Colitis ulcerosa, Diarrhö oder Entzugssymptome, die durch die Beendigung des Gebrauchs von Tabak, Opioiden, Heroin, Kokain oder Morphin, Benzodiazepinen und Benzodiazepin-artigen Arzneimitteln und Alkohol verursacht sind, handelt.

8. 1,4-Diaza-bicyclo[3.2.2]nonan-Derivat nach einem der Ansprüche 1-5, oder ein pharmazeutisch verträgliches Additionssalz davon, zur Verwendung als eine pharmazeutische Zusammensetzung/ein Medikament.

## Revendications

1. Dérivé de 1,4-diaza-bicyclo[3.2.2]nonane représenté par la formule I 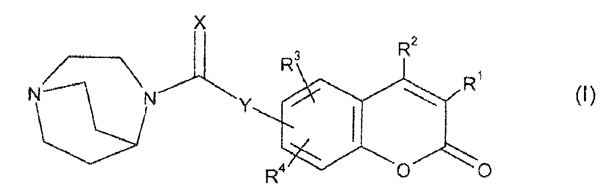 ou un sel pharmaceutiquement acceptable de celui-ci,
dans laquelle
X représente O ;
Y représente O ;
R¹ et R², indépendamment l'un de l'autre, représentent un hydrogène, un halogéno ou un trifluorométhyle ; et
R³ et R⁴ représentent tous les deux un hydrogène.

2. Dérivé de 1,4-diaza-bicyclo[3.2.2]nonane selon la revendication 1, dans lequel l'un parmi R¹ et R² représente un hydrogène, un halogéno ou un trifluorométhyle ; et l'autre parmi R¹ et R², conjointement avec R³ et R⁴, représentent tous un hydrogène.

3. Dérivé de 1,4-diaza-bicyclo[3.2.2]nonane selon la revendication 1, dans lequel R¹ représente un halogéno ; et R², R³ et R⁴ représentent tous un hydrogène.

4. Dérivé de 1,4-diaza-bicyclo[3.2.2]nonane selon la revendication 1, dans lequel R¹, R², R³ et R⁴ représentent tous un hydrogène.

5. Dérivé de 1,4-diaza-bicyclo[3.2.2]nonane selon la revendication 1, qui est
l'ester 2-oxo-2*H*-chromén-7-ylique d'acide 1,4-diaza-bicyclo[3.2.2]nonane-4-carboxylique ;
l'ester 2-oxo-2*H*-chromén-6-ylique d'acide 1,4-diaza-bicyclo[3.2.2]nonane-4-carboxylique ; ou
l'ester 3-bromo-2-oxo-2*H*-chromén-7-ylique d'acide 1,4-diaza-bicyclo[3.2.2]nonane-4-carboxylique ;
ou un sel pharmaceutiquement acceptable de celui-ci.

6. Composition pharmaceutique comprenant une quantité thérapeutiquement efficace du dérivé de 1,4-diaza-bicyclo[3.2.2]nonane selon l'une quelconque des revendications 1 à 5, ou un sel d'addition pharmaceutiquement acceptable de celui-ci, conjointement avec au moins un support ou diluant pharmaceutiquement acceptable.

7. Utilisation du dérivé de 1,4-diaza-bicyclo[3.2.2]nonane selon l'une quelconque des revendications 1 à 5, ou d'un sel d'addition pharmaceutiquement acceptable de celui-ci, pour la fabrication d'une composition pharmaceutique/médicament pour le traitement, la prévention ou l'atténuation d'une maladie ou d'un trouble ou d'une affection d'un mammifère, incluant un être humain, laquelle maladie, lequel trouble ou laquelle affection est un trouble cognitif, un déficit de l'apprentissage, un déficit ou un dysfonctionnement de la mémoire, un syndrome de Down, une maladie d'Alzheimer, un déficit de l'attention, un trouble d'hyperactivité avec déficit de l'attention (THADA), un syndrome de Gilles de la Tourette, une psychose, une dépression, un trouble bipolaire, une manie, une manie-dépression, une schizophrénie, des déficits cognitifs ou de l'attention liés à une schizophrénie, un trouble obsessivo-compulsif (TOC), un trouble panique, un trouble de l'alimentation, une anorexie mentale, une boulimie, une obésité, une narcolepsie, une nociception, une démence liée au SIDA, une démence sénile, un autisme, une maladie de Parkinson, une maladie de Huntington, une sclérose latérale amyotrophique, une anxiété, un trouble d'anxiété non TOC, un trouble convulsif, une épilepsie, un trouble neurodégénératif, une anoxie transitoire, une neurodégénérescence induite, une neuropathie, une neuropathie diabétique, une dyslexie périphérique, une dyskinésie tardive, une hyperkinésie, une douleur légère, une douleur modérée ou sévère, une douleur de caractère aigu, chronique ou récurrent, une douleur provoquée par une migraine, une douleur postopératoire, une douleur de membre fantôme, une douleur inflammatoire, une douleur neuropathique, une céphalée chronique, une douleur centrale, une douleur liée à une neuropathie diabétique, à une algie postzostérienne ou à une lésion de nerf périphérique, un syndrome post-traumatique, une phobie sociale, un trouble du sommeil, une pseudo-démence, un syndrome de Ganser, un syndrome prémenstruel, un syndrome de phase lutéale tardive, une fibromyalgie, un syndrome de fatigue chronique, un mutisme, une trichotillomanie, un décalage horaire, des arythmies, des contractions de muscle lisse, une angine de poitrine, un travail prématuré, une diarrhée, un asthme, une dyskinésie tardive, une hyperkinésie, une éjaculation prématurée, une difficulté d'érection, une hypertension, un trouble inflammatoire, un trouble cutané inflammatoire, une acné, une rosacée, une maladie de Crohn, une maladie intestinale inflammatoire, une recto-colite hémorragique, une diarrhée, ou des symptômes de sevrage provoqués par l'arrêt de l'utilisation de tabac, d'opioïdes, d'héroïne, de cocaïne ou de morphine, de benzodiazépines et de substances médicamenteuses de type benzodiazépine, et d'alcool.

8. Dérivé de 1,4-diaza-bicyclo[3.2.2]nonane selon l'une quelconque des revendications 1 à 5, ou sel d'addition pharmaceutiquement acceptable de celui-ci, pour une utilisation en tant que composition pharmaceutique/médicament.
